# EUROPÄISCHE PATENTSCHRIFT

(11) **EP 1 299 155 B1**
(45) Veröffentlichungstag und Bekanntmachung des Hinweises auf die Patenterteilung: **08.09.2004**
(21) Anmeldenummer: 01956274.3
(22) Anmeldetag: 13.06.2001
(51) Int. Cl.: A61N 5/06, A61M 16/00, A61M 35/00

(54) **THERAPEUTISCHE BESTRAHLUNGSANORDNUNG**
THERAPEUTIC RADIATION ARRANGEMENT
DISPOSITIF DE RAYONNEMENT THERAPEUTIQUE

(30) Priorität: 07.07.2000 DE 10034007
(43) Veröffentlichungstag der Anmeldung: 09.04.2003
(73) Patentinhaber: OptoMed Optomedical Systems GmbH, 12489 Berlin (DE)
(72) Erfinder: WILKENS, Jan, Henrik, 66242 Homburg (DE)
(74) Vertreter: Effert, Bressel und Kollegen
(86) Internationale Anmeldenummer: PCT/DE2001/002215
(87) Internationale Veröffentlichungsnummer: WO 2002/004073

(56) Entgegenhaltungen:
- EP-A- 0 265 082
- WO-A-00/02491
- WO-A-99/39763
- DE-A- 19 852 524
- DE-C- 4 010 301
- DE-U- 29 613 075
- FR-A- 2 660 548
- GB-A- 920 216
- GB-A- 2 024 012
- US-A- 5 084 011
- US-A- 5 865 722
- J. KRUTMANN; H. HÖNIGSMANN: "Handbuch der dermatologischen Phototherapie und Photodiagnostik" , SPRINGER VERLAG , BERLIN XP002187516 in der Anmeldung erwähnt Seite 27

## Beschreibung

Die Erfindung betrifft eine therapeutische Bestrahlungsanordnung, insbesondere zur Behandlung von ganz oder teilweise zellvermittelten Hautentzündungen, wie beispielsweise Neurodermitis, Akne oder Psoriasis.

Therapeutische Bestrahlungsanordnungen sind insbesondere im Bereich der Phototherapie von Hauterkrankungen seit langem bekannt. Je nach Anwendungsgebiet wird dabei ein Patient im Wellenlängenbereich von 315-1500 nm bestrahlt. Insbesondere der Wellenlängenbereich zwischen 315-340 nm (UV-A2) kann nachweislich zu einem erhöhten Krebsrisiko führen, so daß insbesondere bei der Behandlung von atopischen Ekzemen die UV-A1-Therapie (340-400 nm) zur Anwendung kommt.

Die Chemo-Phototherapie als Oberbegriff umfaßt allgemein die Verwendung von optischer Strahlung zur Erzielung von therapeutischen Effekten. Ein Untergebiet der Chemo-Phototherapie ist die sogenannte photodynamische Therapie PDT. Zwei Hauptanwendungsgebiete der PDT sind die Krebsbehandlung und die Behandlung von ganz oder teilweise zellvermittelten Hautentzündungen. Gemeinsames Merkmal der PDT ist die Erzeugung von reaktiven Sauerstoff-Spezies. Hierzu regt die optische Strahlung körpereigene oder inner- oder äußerlich verabreichte Farbstoffmoleküle an, die dann in einen angeregten Zustand überführt werden. Durch Wechselwirkung mit vorhandenen Sauerstoffmolekülen werden durch Energieübertragung reaktive Sauerstoff-Spezies gebildet, die dann die Zelle schädigen oder gar zerstören.

Bei der Krebstherapie mit PDT, wo die Tumorzellen zerstört werden sollen, sind zwei Anwendungsgebiete zu unterscheiden. Der Hauptanwendungsfall ist die Tumorbehandlung innerer Organe. Hierzu wird endoskopisch über eine Glasfaser die optische Strahlung eines Laser zu dem Tumor geleitet und dieser punktuell bestrahlt. Des weiteren werden dem Patienten Photosensibilisatoren gespritzt. Dabei tritt das Problem auf, daß das Tumorgewebe erheblich schlechter durchblutet ist, so daß auch der Sauerstoffgehalt sehr niedrig ist, was aber auch die Umwandlung in reaktive Sauerstoff-Spezies begrenzt.

Daher ist es bei der Tumorbehandlung innerer Organe mit PDT bekannt, dem Patienten inspiratorisch erhöht Sauerstoff zuzuführen, so daß der Sauerstoffgehalt im Tumorgewebe erhöht wird und vermehrt reaktive Sauerstoff-Spezies gebildet werden können. Das zweite Anwendungsgebiet in der Krebstherapie mit PDT ist die Behandlung von äußeren Tumoren wie insbesondere Hautkrebs, wo aufgrund des vorhandenen Sauerstoffs in der Umgebung auf eine zusätzliche Sauerstoffzugabe verzichtet wird.

Bei der Behandlung von ganz oder teilweise zellvermittelten Hautentzündungen handelt es sich im Gegensatz zu Tumoren stets um großflächige Entzündungen, so daß hier flächenhafte Strahlungsquellen zur Anwendung kommen, die Behandlungsflächen von 5cm² bis 2m² simultan bestrahlen. Ein weiterer Unterschied zu den Tumoren ist die erhöhte Durchblutung von Entzündungen, die bereits äußerlich durch die vorhandene Rötung im Entzündungsbereich gut erkennbar ist.

Das überwiegend in der Behandlung von ganz oder teilweise zellvermittelten Hauterkrankungen eingesetzte Verfahren der PDT ist die hochdosierte UV-A1-Therapie im Wellenlängenbereich von 340-400 nm. Dabei ist zum einen die Abgabe hoher Dosen von beispielsweise 130 J/cm² und zum anderen die Bestrahlungsstärke von mehr als 60 mW/cm² erforderlich, um ausreichende therapeutische Effekte zu bewirken. Trotzdem bleibt bei ca. 20 - 30 % der behandelten Patienten die UV-A1-Therapie wirkungslos.

Zusätzlich ist es möglich, in haarlosen Skh-hrl -Albinomäusen durch hohe kumulative UV-A1-Dosen epitheliale Hauttumore zu induzieren. Es kann daher zur Zeit nicht ausgeschlossen werden, daß der unkritische Einsatz der UV-A1-Therapie zu einer Erhöhung des Hautkrebsrisikos führt. Ebenso ernst zu nehmen sind tierexperimentelle Untersuchungen, die darauf hinweisen, daß der Wellenlängenbereich, der für die Entstehung von malignen Melanomen verantwortlich ist, vor allen der UV-A-Strahlung entspricht. Die Anwendung der hochdosierten UV-A1-Therapie als Dauertherapie bei der atopischen Dermatitis erscheint deshalb zur Zeit als unverantwortbar.

Darüber hinaus wird die atopische Dermatitis auch mit Hilfe der systemischen Phototherapie d. h. der oralen Gabe von Psoralenen mit einer anschließenden Ganzkörperbestrahlung mit einer Breitband-UV-A1 -Bestrahlung kombiniert. Im Gegensatz zur PDT kommt es hier zu einer direkten DNS-Schädigung der Zellen durch das Psoralen ohne Zwischenschaltung von Sauerstoff. Trotz der unbestrittenen Effektivität der PUVA-Therapie bei atopischen Ekzemen darf nicht übersehen werden, daß sie bei dieser Indikation mit einer Reihe schwerwiegender Nachteile behaftet ist. Es ist eine hohe Anzahl der Behandlungen zum Abheilen des atopischen Ekzems notwendig. Oft ist es erforderlich, die Erhaltungstherapie über viele Monate und Jahre fortzuführen. Die Erkrankung beginnt häufig im Kindes- und Jugendalter. Der therapeutischen Effektivität der PUVA-Langzeittherapie des Ekzems steht daher das bei langfristiger Anwendung erhöhte Hautkrebsrisiko als eine gewichtige Nebenwirkung gegenüber. Ein weiteres Problem stellen die relativ hohen Bestrahlungsstärken dar, die einen entsprechend hohen apparativen Aufwand erfordern. Einen guten Überblick über den Stand der Technik der Phototherapie bei T-zellvermittelten Hauterkrankungen liefert das "Handbuch der dermatologischen Phototherapie und Photodiagnostik, J. Krutmann, H. Königsmann, Springer Verlag 1997, S. 68-83 m.w.N.".

Aus WO 00/02491-A ist bereits eine Bestrahlungsanordnung bekannt gemäß dem Oberbegriff des Anspruchs 1.

Der Erfindung liegt daher das technische Problem zugrunde, eine therapeutische Bestrahlungsanordnung insbesondere zur Behandlung von ganz oder teilweise zellvermittelten Hautentzündungen zu schaffen, die eine verbesserte therapeutische Effektivität aufweist.

Die Lösung des technischen Problems ergibt sich durch den Gegenstand mit den Merkmalen des Patentanspruchs 1. Weitere vorteilhafte Ausgestaltungen der Erfindung ergeben sich aus den Unteransprüchen.

Hierzu umfaßt die Bestrahlungsanordnung eine Einrichtung zur inspiratorischen und topischen Sauerstoffzugabe. Dadurch wird der Sauerstoffgehalt in den zu bestrahlenden Körperpartien erhöht, was wiederum die Bildung von reaktiven O₂-Spezies verbessert.

### Die Erfindung geht dabei von folgenden Erkenntnissen aus:

Der therapeutische Effekt bei der Phototherapie beruht auf einer Anregung von Farbstoff-Molekülen, sogenannten Porphyrinen, deren Anregung durch Energieübertragung den vorhandenen Sauerstoff in reaktive O₂-Spezies umwandelt, die wiederum lokalisationsabhängige Schäden wie beispielsweise Schädigungen der Plasmamembran, der Lysomen oder Mitochondrien verursachen.

Dadurch ergibt sich eine Apoptose von T-Lymphozyten aber auch eine Abtötung von Sporen, Pilzen und Hautkeimen. Die Wirkung einer derartigen Phototherapie beruht danach auf drei Faktoren, nämlich:
1) der Menge an verfügbarem Licht
2) der Menge der verfügbaren Porphyrine und
3) der Menge der verfügbaren Sauerstoffmoleküle.

Dabei wird die Menge an im Körper verfügbarem Licht durch die Bestrahlungsstärke bzw. die Dosis und die Menge der verfügbaren Porphyrine durch zusätzliche Photosensibilisatoren erhöht. Aufgrund der im Regelfall starken Rötung ist man bisher davon ausgegangen, daß die zu behandelnden Körperpartien stark durchblutet und ausreichend mit Sauerstoff versorgt sind. Dies ist jedoch nicht der Fall. Vielmehr verursacht die Entzündung bei ganz oder teilweise zellvermittelten Hautentzündungen selbst eine Ansäuerung im betroffenen Gewebe. Die damit verbundene pH-Verschiebung verschlechtert trotz der erhöhten Durchblutung die Sauerstoffversorgung des Gewebes durch Verminderung der Transportkapazität von Sauerstoffmolekülen wie beispielsweise Hämoglobin. Darüber hinaus kommt es über eine Photorelaxation zu einer Erschlaffung von präkapillaren Sphinktern im subdermalen Gefäßplexus, was den transkutanen Sauerstoffdruck erniedrigt. Somit stellt die verfügbare Sauerstoffkonzentration einen entscheidenden Parameter für die Wirksamkeit der PDT bei der Behandlung von ganz oder teilweise zellvermittelten Hautentzündungen dar, wobei entgegen allen bisherigen Annahmen die normal vorhandene Sauerstoffmenge nicht ausreichend groß ist. Diese wird durch die inspiratorische und topische Sauerstoffzugabe entscheidend angehoben, so daß erhöht Sauerstoffmoleküle zur Verfügung stehen, die in reaktive O₂-Spezies umwandelbar sind.

Deshalb verursacht additiver Sauerstoff eine Wirkungsverstärkung, die entweder zur Wirkungsverstärkung oder Verminderung der eingestrahlten Leistung und des damit verbundenen apparativen Aufbaus eingesetzt werden kann. Der normalerweise bei einem Gesunden vorhandene arterielle Sauerstoffpartialdruck beträgt ca. 12 kPa und wird durch Inhalation von reinem Sauerstoff auf über 67 kPa angehoben. Diese hohe Sauerstoffkonzentration führt nun zur Bildung von mehr Singulettsauerstoff und damit neben der Apoptose von T-Lymphozyten zur Abtötung von Sporen, Pilzen und Hautkeimen, wie beispielsweise Staphylococcus aureus, der bis zu 90% aller Patienten mit Neurodermitis befallen hat. Bei der Behandlung von Pilzen, z.B. Fußpilz, ist diese Behandlung besonders effizient, da das Fußpilzwachstum teilweise in bradytropen Geweben, z.B. in den Nagelgeweben, stattfindet, so daß es günstig ist, per Diffusion dort einen möglichst hohen O₂-Gradienten auszubilden. Da das Diffusionshemmnis eines Nagels wesentlich größer ist als das der Hornhaut, werden mittels eines Ultraschall-Applikators Gewebekavitationen erzeugt, die die Diffusion von Sauerstoff um den Faktor 10 erhöhen. Die Verwendung eines Ultraschall-Applikators ist dabei nicht nur auf die Behandlung von Fußpilz beschränkt, sondern kommt allgemein zur Erhöhung der topischen Sauerstoffzufuhr zur Anwendung.

Ein weiteres Anwendungsgebiet der therapeutischen Bestrahlungsanordnung ist die Behandlung von Autoimmunerkrankungen mit rezirkulierenden Lymphozyten wie beispielsweise Arteriosklerose, Multiple Sklerose, Rheuma, Asthma, COLD, Transplantatabstoßung und verschiedene Bindegewebs- und Organerkrankungen. Dabei wird zum einen die Erkenntnis ausgenutzt, daß innerhalb der aktivierten Lymphozyten ein extremes Sauerstoffgefälle, obwohl diese im zum Teil sehr sauerstoffreichem Blut sich befinden, so daß bisher nur begrenzte photoinduzierte Effekte erreichbar waren. Die Ursache hierfür kann entweder in einem erhöhten Sauerstoffverbrauch liegen und/oder in einer starken Verminderung der Sauerstoffdiffusion im Zytosol. Dieser Sauerstoffmangel wird nun durch die inspiratorische und/oder topische Sauerstoffzugabe behoben. Die andere Erkenntnis ist, daß jeweils ein Teil der rezirkulierenden Lymphozyten sich oberflächlich im Bereich der Haut befindet und somit von der optischen Strahlung erreicht werden kann, obwohl die "Quelle" tief im inneren des Körpers sich befinden kann. Durch die partielle Deaktivierung sind jedoch erhebliche therapeutische Erfolge erzielbar, vergleichbar der Photopherese, wo dem Patienten geringe Plasmamengen entnommen werden und nach Behandlung mit Psoralen und UVA wieder zugeführt werden.

Aufgrund der bisherigen Erkenntnisse scheint die Bestrahlungsanordnung darüber hinaus prinzipiell zur Inaktivierung von HIV-Viren geeignet zu sein. Bisher ist es bereits bekannt, daß mit Hilfe des Benzoporphyrins BPD und einer Lichtbehandlung sowohl das vesikuläre Stromatitis-Virus als auch das felline Leukämievirus behandelt werden konnten. Auch das HIV-Virus, selbst in der HCT-resistenten Form, führte zu einer Nicht-Infektiösität von infiziösem Material, wobei Singulett-Sauerstoff für die irreversible Schädigung der Virushülle verantwortlich gemacht wird. Die proliferierenden Zellen, die aktiv HIV-Viren replizieren, werden daher bevorzugt vernichtet, während latent infizierte Zellen der Photodestruktion entkommen. Es ist somit möglich, den höheren Porphyrinspiegel in den aktiv replizierenden Zellen für deren Ausschaltung auszunutzen. Hierzu bedarf es jedoch noch weiterer klinischer Studien.

In einer weiteren bevorzugten Ausführungsform umfaßt die Einrichtung einen gasdichten Hüllkörper, der je nach Anwendung ausgebildet ist, insbesondere bei lokalen, oberflächlichen Entzündungen kann der Hüllkörper als Glocke ausgebildet sein, der dann lokal auf die zu behandelnde Stelle aufgesetzt und mit Sauerstoff geflutet wird. Besonders vorteilhaft ist jedoch ein Gehäuse, in dem der Patient vollständig liegt oder steht. Anschließend wird kurzzeitig das Gehäuse mit Sauerstoff geflutet und die darin befindliche Luft ausgetauscht. Der Patient atmet dann reinen Sauerstoff bzw. ein Sauerstoffgemisch ein, wobei gleichzeitig dessen ganze Haut umspült wird. Vorzugsweise wird in diesen Fällen die Bestrahlungsquelle innerhalb des Gehäuses angeordnet. Danach werden zum einen die Reflexions- und Absorptionsverluste am Hüllkörper vermieden und zum anderen kann die Bestrahlungsquelle näher am Patienten angeordnet werden, so daß sich höhere Bestrahlungsstärken einstellen. Unter Umständen kann es vorteilhaft sein, der gasdichten Bestrahlungskammer hyperbaren Sauerstoff zuzuführen, um bei besonders ausgeprägten Fällen eine ausreichende Oxygenierung zu erreichen.

Bei Anwendungen mit lokalen Hüllkörpern, kann die Einrichtung zur inspiratorischen Sauerstoffzugabe als Nasen-Mund-Maske mit angeschlossener Sauerstoffflasche ausgebildet sein. Dabei ist die Nasen-Mund-Maske vorzugsweise aus einem transparenten Material für die emittierende Strahlung ausgebildet, so daß gegebenenfalls entzündete Stellen in diesem Bereich ebenfalls behandelbar sind.

Eine weitere Möglichkeit zur topischen Sauerstoffzufuhr ist die Verwendung sauerstoffgesättigter Emulsionen. Hierzu umfaßt die Emulsion zwei Substanzen mit unterschiedlicher Löslichkeit für Sauerstoff. Die eine Substanz weist dabei eine sehr gute Löslichkeit für Sauerstoff auf. Die andere Substanz hingegen bildet die Trägersubstanz und weist eine niedrige Sauerstofflöslichkeit und gegebenenfalls eine niedrige Diffusionskonstante für Sauerstoff auf. Unter geringer Sauerstofflöslichkeit werden dabei Löslichkeiten von 0,01-1 ml Sauerstoff / 100 ml Substanz bei 10⁵ Pa verstanden. Unter hoher Sauerstofflöslichkeit werden dabei Löslichkeiten von 3-300 ml Sauerstoff / 100 ml Substanz bei 10⁵ Pa verstanden. Vorzugsweise weist darüber hinaus die Trägersubstanz eine eine hohe Viskosität im Bereich zwischen 0,5 bis 10 Stokes auf.

Zur Herstellung der sauerstoffgesättigten Emulsion werden die beiden Substanzen unter Sauerstoffüberdruck von beispielsweise 1-2* 10⁶ Pa entweder mechanisch oder mittels Ultraschall gemixt. Als Trägersubstanzen kommen beispielsweise Glycerin, Paraffine, Fette und Öle in Frage. Als Sauerstoffträger eignen sich beispielsweise perfluorchemische Derivate oder verschiedene Polymere wie beispielsweise Polypropylen, PMMA oder Silikonpolymere wie Polydimethylsiloxane.

Die bevorzugte Größe der Sauerstoffträgerteilchen liegt dabei zwischen 0,1-10 µm. Nach Aufbringung der Emulsion auf die behandelnde Fläche unter Normaldruck entstehen dennoch keine Bläschen, obwohl eine sehr hohe Gaskonzentration innerhalb der Tröpfchen oder Partikel vorliegt. Die Trägersubstanz der Tröpfchen oder Partikel bleibt trotz der hohen Gaspartialdrucke stabil, weil die Trägersubstanz nur über eine geringe Gaslöslichkeit verfügt und nach der hydrostatischen Kompression auch keine Kavitationskerne für die Entstehung von Gasbläschen mehr vorhanden sind. Der niedrige Gasdiffusionskoeffizient in der Trägersubstanz bedingt eine langsame Gasfreisetzung sowohl aus den Tröpfchen oder Partikeln in die Trägersubstanz als auch von der Emulsion in die sauerstoffarme Umgebung. Obwohl hier nur eine relativ niedrige Konzentration in der Trägersubstanz vorliegt, bedingt der gleichzeitig vorhandene sehr hohe Partialdruck von Sauerstoff in der Emulsion einen hohen Konzentrationsgradienten zum hypoxischen Gewebebereich. Aufgrund der hohen Viskosität der Trägersubstanzen müssen diese aus einem gasdichten Behälter unter Druck appliziert werden, wie dies beispielsweise analog für Silikonmischungen bekannt ist. Weiter sollte der gasdichte Behälter über eine Ladeventil verfügen, um während des Nichtgebrauchs den Sauerstoffdruck konstant zu halten.

Weiter erscheint es möglich die Silikonderivate, insbesondere Polysiloxane auch ohne Trägersubstanz zu verwenden, da diese von Hause aus eine sehr gute Sauerstofflöslichkeit bei einer mittleren Viskosität von 0,5-10 Stokes aufweisen. Bei Verwendung von 2*10⁶ Pa werden ca. 2000 ml Sauerstoff in 100 ml Polysiloxan gelöst, wobei aufgrund der hohen Viskosität Microbubbles und Kavitationen unter Rekompressionsbedingungen nicht auftreten. Entsprechend dem Henryschen Gesetz beträgt die Sauerstoffkonzentration an der Grenzschicht zur Hautoberfläche nach Auftragung des beladenen Polysiloxans bis zu 20 ml Sauerstoff pro Gramm Trägersubstanz. Unter Annahme einer Sauerstofflöslichkeit der Haut von 4*10⁻⁵ g Sauerstoff pro Gramm Gewebe ergibt sich ein Sauerstoffpartialdruck von bis zu 10⁸ Pa. Aufgrund der Diffusibilität ist mit einem erheblichen intrakutanen Gradienten zu rechnen. Da im Gegensatz zur Emulsion hier auf eine Trägersubstanz verzichtet werden kann, steht die gesamte Sauerstoffbindungskapazität grenzflächennah zur Verfügung.

In einer weiteren bevorzugten Ausführungsform kann die Einrichtung zur topischen Sauerstoffzufuhr als eine beschichtete und mit Sauerstoff beladene Polymerfolie ausgebildet sein, die als Barriere zwischen Patienten und einem Wasserfilter eingesetzt wird. Vorzugsweise ist die Polymerfolie als Silikonpolymerfolie ausgebildet, die im Wellenlängenbereich der Strahlungsquellen transparent ist. Die Polymerfolie wird dabei über bekannte Oberflächenbeschichtungsverfahren mit einer sauerstoffundurchlässigen Barriere an der hautabgewandten Seite beschichtet. Hierzu kann beispielsweise eine Polyäthylenfolie auf die Silikonpolymerfolie aufgebracht werden, deren Sauerstofflöslichkeit Größenordnungen unter der Silikonpolymerfolie liegt.

Vorzugsweise setzt die inspiratorische und/oder topische Sauerstoffzufuhr zeitlich etwas vor dem Beginn der Bestrahlung ein. Hierdurch konnten insbesondere bei stark ausgeprägten Entzündungsreaktionen bessere Ergebnisse erzielt werden. Je nach Ausprägung des Entzündungsbildes kann es günstig sein, die Sauerstoffgabe bereits mehre Stunden vor der Phototherapie zu beginnen. Die Zeiträume können sich dabei von einer Minute bis zu 24 Stunden erstrecken. In Einzelfällen wurden Patienten auch mit Sauerstoffkonzentratoren bzw. tragbaren Flüssigsauerstoffgeräten während der gesamten Behandlung mit Sauerstoff versorgt. Hierdurch wird sichergestellt, daß das zu behandelnde Gewebe zum Beginn der Behandlung ausreichend mit Sauerstoff versorgt wird, um eine maximale Singulet-Sauerstoff-Quantenausbeute zu erzielen. Wegen der individuell stark schwankenden Entzündungsintensität und den hiermit verbundenen starken Schwankungen sowohl der Sauerstoffkonzentration als auch der Sauerstoffdiffusionskonstanten kann es erforderlich sein, sehr lange Vorlaufzeiten zu wählen. In der Regel sind jedoch Vorlaufzeiten zwischen 15-60 Minuten ausreichend.

In einer weiteren bevorzugten Ausführungsform umfaßt die Bestrahlungsanordnung eine Kühleinrichtung für die zu behandelnde Fläche. Dabei wird vorzugsweise mittels der Kühleinrichtung die zu bestrahlende Fläche unter die Normaltemperatur gekühlt. Sehr gute Ergebnisse wurden dabei mit lokalen Temperaturen im Bereich von <30°C erreicht. Vorzugsweise wird die Temperatur auf 10-20°C abgesenkt. Durch die Abkühlung kommt es zu einer Verringerung der Stoffwechselaktivität, wodurch der Sauerstoffverbrauch reduziert wird, so daß eine erhöhte Sauerstoffkonzentration in den zu behandelnden Geweben aufgebaut werden kann. Insbesondere bei Ganzkörperbestrahlungen wird die Kühleinrichtung durch eine Wasserlage gebildet, in der dann der Patient liegt, wobei die Wassertemperatur jeweils einige Grad unter der angestrebten Hauttemperatur liegt.

Wie bereits ausgeführt ist ein weiterer wichtiger Parameter für die Effizienz der PDT die Menge des vorhandenen Porphyrins. Da die natürlichen Porphyrine nicht sehr photostabil und nur in geringen Konzentrationen vorhanden sind, werden vorzugsweise zusätzlich Photosensibilisatoren äußerlich in Form von Salben, Liposomenpräparationen und/oder Emulsionen auf die zu behandelnde Fläche aufgetragen. Beispiele für derartige Photosensibilisatoren sind Hämatoporphinderivate, n-THPC, Porphycene, Sn-Etioporphyrin, NPE6, Zn-Phthalocyanin, Benzoporphyrin, Pro-Drugs, wie beispielsweise 5-delta-Amino-Laevulinsäure, Phäophorbid und Derivate, Methylenblau, Bengalrosa oder Texaphyrine. Dabei hängt die Wahl des Photosensibilisators von dem Spektrum der Bestrahlungsquelle ab.

Als ein besonders effektiver Zusatzstoff hat sich Glukose erwiesen, der sehr breitbandig verwendet werden kann und sehr stabil ist. Glukose führt nämlich ausschließlich in Anwesenheit von Sauerstoff zu einer drastischen Verschiebung des metabolischen Profils der endogenen Synthese von Porphyrinderivaten. Darüber hinaus kommt es zu einer allgemeinen Synthesesteigerung von Porphyrinen. Das endogen gebildete Protoporphyrin IX ist ein mitochondriales Produkt, das bei Photoaktivierung die mitochondrialen Membranen schädigt. Durch die Einwirkung von Glukose vermehrt entstehenden Photooxydationsprodukte aus der Klasse der sog. Photoporphyrine zeigen ein leicht geändertes Absorptionsverhalten und sind erheblich photostabiler als das PP IX, bei dem es sich um eine extrem photolabile Substanz handelt, die vor allem bei hohen Flächenleistungen innerhalb weniger Minuten durch Photobleaching metabolisiert wird. Als besonders geeignet haben sich Glukose-Glycerin-Emulsionen bzw. Glukose-Liposomen erwiesen.

Zur weiteren Erhöhung der Effizienz können den Photosensibilisatoren und insbesonder den Glukose-Verbindungen Chelatbildner (EDTA) zugefügt werden, die Eisen in lösbaren Komplexen binden, wodurch eine Verschiebung der Metabolisierung von unwirksamen Hämoglobin zum Photoporphyrin IX erreicht wird.

Vorzugsweise erzeugen die Strahlungsquellen eine Strahlungsleistungsdichte von größer 20 mW/cm² , da patientenspezifische Schwellwerte für die Wirksamkeit nicht auszuschließen sind.

Da, wie bereits ausgeführt, der UV-Bereich hinsichtlich seiner photobiologischen Wirkungen äußerst kritisch ist, wird dieser vorzugsweise völlig bzw. soweit als möglich unterdrückt. Daher wird bevorzugt der Bereich von 400-440 nm verwendet. Neben dem Wellenlängenbereich von 400-440 nm sind auch noch die Wellenlängenbereiche um 540 nm bzw. 600-700 nm von besonderem Interesse, da dort die Porphyrinderivate und ihre Photoprodukte Nebenmaxima im spektralen Absorptionsverhalten aufweisen.

In einer weiteren bevorzugten Ausführungsform wird daher eine Strahlungsquelle verwendet, die im Bereich von 400-440 nm und 530-570 nm die maximale Leistungsdichte aufweist. Dabei wird unter maximaler Leistungsdichte verstanden, daß mindestens 80% der spektralen Leistungsdichte der Nutzstrahlung in diesen Wellenlängenbereichen von den Strahlungsquellen emittiert werden. Der Vorteil dieser Wellenlängenbereiche ist deren Ungefährlichkeit im Vergleich zu UV-A1 hinsichtlich der Karzinogenität und Photoelastose bzw.Hautalterung und die relativ hohe spektrale Absorption der Photoporphyrine in diesem Bereich. Eine Anzahl geeigneter Strahlungsquellen in diesem Wellenlängenbereich sind in der DE 198 52 524 A1 aufgeführt. Beispielsweise ist die Strahlungsquelle als Quecksilber-Niederdruckentladungslampe mit einem der nachfolgenden Leuchtstoffe Sr₂P₂O₇:Eu, (SrMg)₂P₂O₇:Eu, Sr₅Cl(PO)₄)₃:Eu, BaMg₂Al₁₆O₂₇:Eu, SrMgAl₁₈O₃₉:Eu, BaMg₂Al₁₆O₂₇:Eu:Mn, Sr₃(PO₄)₂:Eu, Ba₃(PO₄)₂:Eu, CaWO₄:Pb oder CaWO₄ ausgebildet.

Höhere Leistungen lassen sich jedoch mit Mittel- und Hochdrucklampen erzeugen. Daher wird in einer bevorzugten Ausführungsform eine Quecksilbermitteldrucklampe mit Argon als Zündgas sowie Zusätzen von Gallium und Galliumhalogeniden, insbesondere Gallium-Jodid verwendet. Die UVA- und UVB-Anteile werden durch geeignte Filter unterdrückt. Das Gewichtsverhältnis von Gallium zu Gallium-Jodid und Quecksilber ist dabei vorzugsweise 1/2,4/180. Diese Strahlungsquelle weist dann weit über 80% ihrer spektralen Leistungsdichte in den Wellenlängenbereichen 400-440 und 530-570 nm auf. In den klinischen Versuchen wird derzeit eine derartige Bestrahlungsquelle mit Bestrahlungsstärken über 300 mW/cm², verwendet mittels derer 200 J/cm² Tagesdosis zur Behandlung von Neurodermitis verabreicht wird. Trotz minimaler Anteile im Bereich zwischen 397-400 nm ist eine derartige Strahlungsquelle gemäß der Grenzwerte der ACGIH und ICNIRP als UV-frei zu bezeichnen. Als alternative oder ergänzende Lampenadditive kommen Indium-Jodid, Selen, Antimon, Zink und Kadmium in Frage. Aufgrund der geringeren spektralen Absorption im Wellenlängenbereich von 530-570 nm im Vergleich zu 400-440 nm kann dieser Wellenlängenbereich gegebenenfalls auch unterdrückt werden, um die verbleibende spektrale Leistungsdichte auf den hochwirksamen Bereich von 400-440 nm zu konzentrieren.

In einer weiteren bevorzugten Ausführungsform werden die flächenhaften Strahlungsquellen als Natrium-Dampflampen ausgebildet, die mit einem erhöhtem Dampfdruck betrieben werden. Der erhöhte Dampfdruck kann entweder durch einen Überlastbetrieb oder durch thermische Rückkopplung mittels Reflektoren erreicht werden. Als Reflektoren kommen dabei insbesondere Spaltreflektoren in Betracht, die im einfachsten Fall aus einer Aluminium-Folie bestehen, die über die volle Länge um die Natrium-Dampflampe gewickelt wird und einen Öffnungswinkel von 20-180° freiläßt. Aufgrund des erhöhten Dampfdrucks kommt es zu einer Verbreiterung der beiden Flanken rechts und links von der Selbstinversionslinie um 590 nm. Der Bereich von 620-650 nm hat unter normalen Betriebsbedingungen einen relativen spektralen Emissionsanteil von ca. 7%, der sich bei einem Betrieb mit der 2,5 fachen Nennleistung verdoppelt. Da Natrium-Dampflampen äußerst preisgünstig sind, läßt sich dadurch eine sehr preiswerte Strahlungsquelle im Bereich von 620-650 nm realisieren. Der Vorteil dieses Wellenlängenbereiches gegenüber kurzwelligen Strahlungsquellen ist die größere Eindringtiefe, so daß beispielsweise zellvermittelte Erkrankungen der Haarwurzeln wie Alopecia areata in der lokalen oder Ganzkörperform behandelbar sind.

Die Erfindung wird nachfolgend anhand eines bevorzugten Ausführungsbeispiels näher erläutert. Die Fig. zeigen:
- Fig.1: eine Vorderansicht einer therapeutischen Bestrahlungsanordnung mit topischer und inspiratorischer Sauerstoffzufuhr,
- Fig.2: eine Vorderansicht einer einer therapeutischen Bestrahlungsanordnung mit inspiratorischer Sauerstoffzufuhr,
- Fig.3: eine schematische Darstellung einer Einrichtung zur topischen Sauerstoffzugabe mittels einer Emulsion und
- Fig.4: eine Vorderansicht einer therapeutischen Bestrahlungsanordnung mit inspiratorischer Sauerstoffzufuhr.

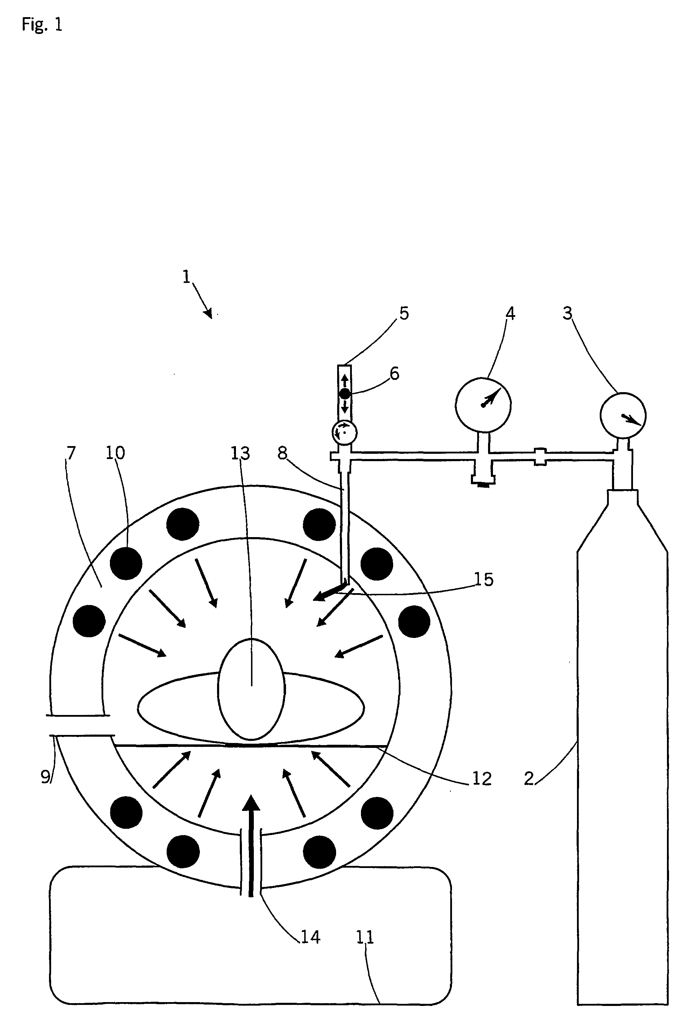

In der Fig.1 ist schematisch eine therapeutische Bestrahlungsanordnung 1 dargestellt. Die Bestrahlungsanordnung 1 umfaßt eine Sauerstoffflasche 2, ein Hochdruckventil 3, ein Manometer 4, einen Durchflußregler 5 mit Steigkugel 6, einen Hüllkörper 7 mit einer Eintrittsöffnung 8 und einer Austrittsöffnung 9, eine Vielzahl von optischen Strahlungsquellen 10 und ein Kühlaggregat 11. In dem Hüllkörper 7 ist eine netzartige Auflage 12 angeordnet, auf der ein Patient 13 liegen kann. Des weiteren ist der Hüllkörper 7 mit einer Kühlluftzuführung 14 ausgebildet, über die Kühlluft vom Kühlaggregat 11 in den Hüllkörper eingeströmt werden kann. Der Hüllkörper 7 ist bis auf die zuvor beschriebenen Ein- und Austrittsöffnungen gasdicht ausgebildet.

Zur Behandlung legt sich der Patient 13 auf die netzartige Auflage 12, wozu der Hüllkörper 7 beispielsweise aufschwenkbar ausgebildet ist. Anschließend wird das Hochdruckventil 3 geöffnet und Sauerstoff 15 strömt über die Eintrittsöffnung 8 in den Hüllkörper 7, wobei über den Durchflußregler 5 eine Feinregelung der Durchflußmenge vorgenommen werden kann. Somit befindet sich der Patient 13 in einer Sauerstoffatmosphäre. Durch Atmung kommt es somit zu einer inspiratorischen Sauerstoffaufnahme. Gleichzeitig wird der Körper des Patienten von Sauerstoff umspült, so daß es durch Hautdiffusion ebenfalls zu einer topischen Sauerstoffaufnahme kommt. Nachdem der Hüllkörper 7 mit Sauerstoff 15 geflutet wurde, werden die Strahlungsquellen 10 eingeschaltet und bestrahlen die zubehandelnden Körperpartien. Zur notwendigen Kühlung des Patienten 13 wird über die Kühluftzuführung 14 gekühlte Luft in den Hüllkörper 7 geblasen. Zur Erreichung einer gleichmäßigen Kühlung des Patienten 13 wird dabei die Kühlluft durch Ausgestaltung der Kühlluftzuführung 14 oder durch andere Maßnahmen im Hüllkörper 7 verwirbelt. Über die Austrittsöffnung 9 wird dabei ein Druckausgleich erreicht. Neben diesem offenen System ist es auch denkbar, einen geschlossenen Saustoffkreislauf einzurichten, wobei das austretende Gas entfeuchtet, von Kohlendioxid gereinigt und zurückgeführt werden müßte.

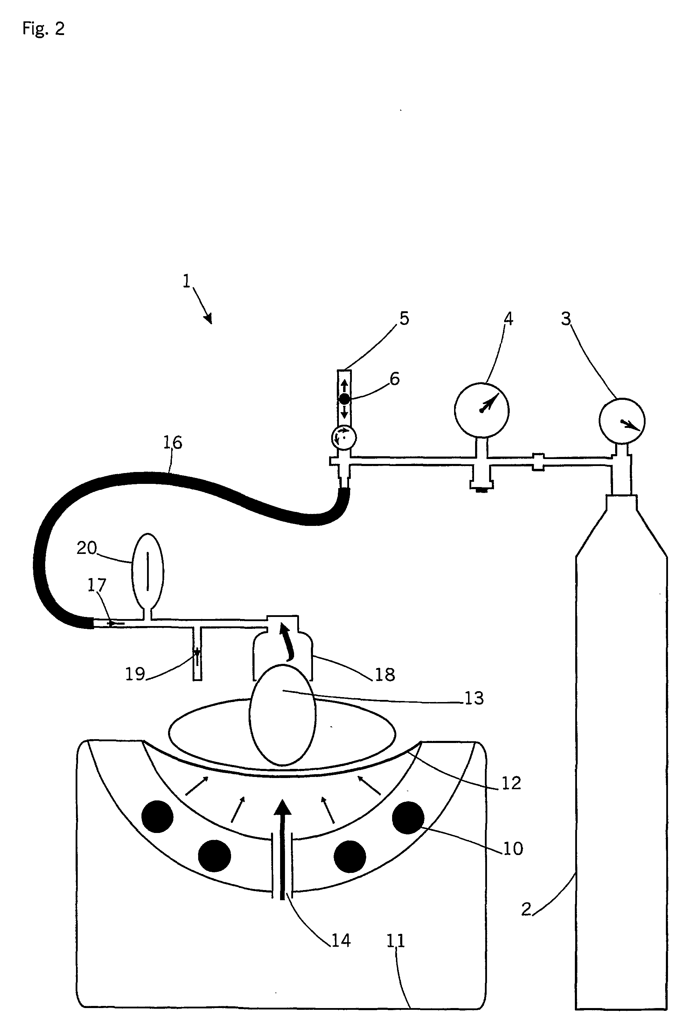

In der Fig. 2 ist eine Bestrahlungsanordnung 1 mit einer reinen inspiratorischen Sauerstoffzugabe dargestellt. Dabei liegt der Patient 13 wieder auf einer netzartigen Auflage 12. Im Gegensatz zur Bestrahlungsanordnung gemäß Fig.1 ist hinter dem Durchflußregler 5 an einer Gasaustrittsöffnung ein Schlauch 16 angeordnet, der über ein Einatemventil 17 mit einer Mund-Nasenmaske 18 verbunden ist. Zwischen Einatemventil 17 und Mund-Nasenmaske 18 ist ein Ausatemventil 19 und ein Reservoirbeutel 20 angeordnet. Zur Behandlung wird dem Patienten 13 die Nasen-Mundmaske aufgesetzt und das Hochdruckventil 3 geöffnet, so daß der Patient reinen Sauerstoff einatmet und dessen ausgeatmete Luft über das Ausatemventil 19 abgeführt wird.

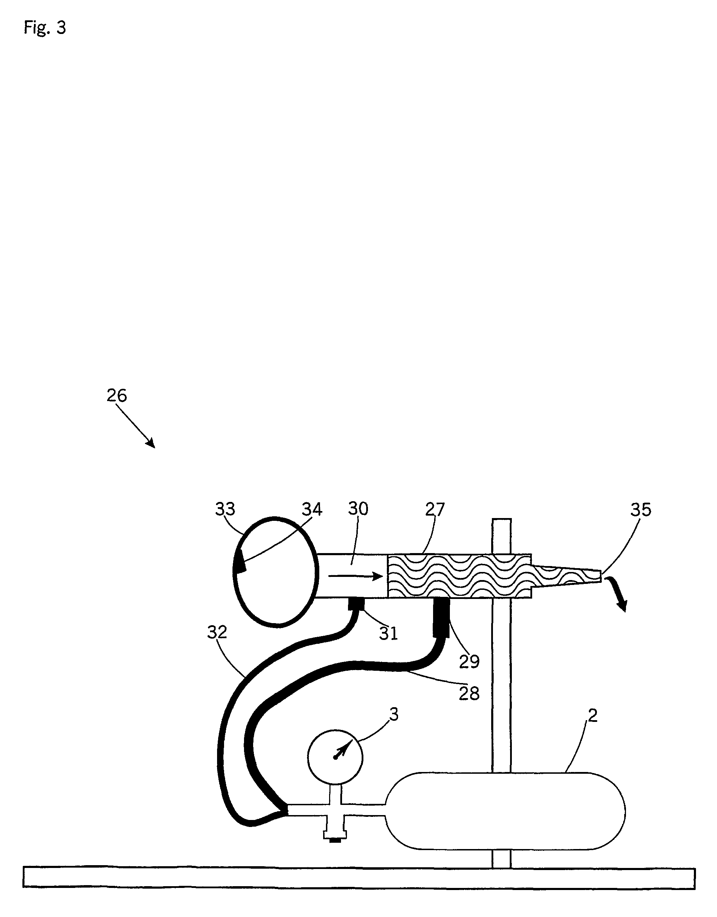

In der Fig. 3 ist eine Einrichtung 26 zur topischen Sauerstoffzuführung mit einer Emulsion dargestellt. Die Einrichtung 26 umfaßt einen gasdichten Behälter 27, in dem sich eine Emulsion aus mindestens zwei Substanzen befindet, wobei die eine Substanz eine geringe Sauerstofflöslichkeit und eine geringe Sauerstoffdiffusionskonstante aufweist und die andere Substanz eine hohe Sauerstofflöslichkeit aufweist und mit Sauerstoff beladen ist. Des weiteren umfaßt die Einrichtung 26 eine Sauerstoffflasche 2, ein Hochdruckventil 3 und einen ersten Schlauch 28, über den die Sauerstoffflasche 2 durch ein Ladeventil 29 mit dem gasdichten Behälter 27 verbunden ist, so daß die Emulsion kontinuierlich mit Sauerstoff beladen werden kann. Dem gasdichten Behälter 27 ist ein Stempel 30 zugeordnet, der durch Überdrück in Pfeilrichtung bewegbar ist. Hierzu ist dem Stempel 30 ein Einlaßventil 31 zugeordnet, das über einen zweiten Schlauch 32 mit dem Hochdruckventil 3 der Sauerstoffflasche 2 verbunden ist. Hinter dem Stempel 30 ist ein Handgriff 33 mit einem Schalter 34 angeordnet, über den das Einlaßventil 31 betätigbar ist. Zur Entnahme der Emulsion, die dann zur topischen Sauerstoffzuführung auf die Haut des Patienten aufgetragen werden soll, wird der Schalter 34 betätigt und das Einlaßventil 31 geöffnet. Unter Überdruck strömt dann der Sauerstoff durch den zweiten Schlauch 32 in den Stempel 30, wodurch die Emulsion aus einer Öffnung 35 blasenfrei gepreßt wird.

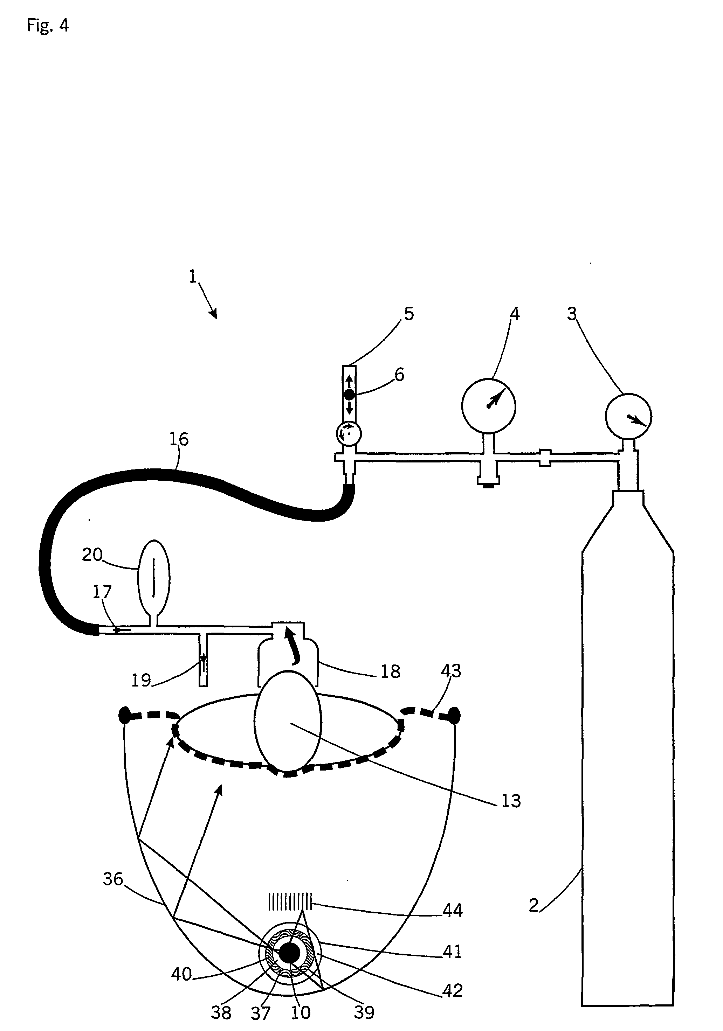

In der Fig.4 ist eine alternative Ausführungsform der therapeutischen Bestrahlungsanordnung 1 mit inspiratorischer Sauerstoffzufuhr dargestellt. Dabei entsprechen die Teile der inspiratorischen Sauerstoffzufuhr denen aus Fig.2 und sind mit gleichen Bezugszeichen dargestellt. Die Bestrahlungsanordnung 1 umfaßt eine wassergefüllte paraboloide Reflektorrinne 36, die beispielsweise mit einem hochreflektivem Lack auf der Basis AlO₃ oder TiO₂ beschichtet ist. Alternativ kann auch ein interferenzbeschichteter Aluminium-Reflektor verwendet werden, der auf den Wellenlängenbereich der Strahlungsquellen 10 abgestimmt ist. Die Strahlungsquellen 10, die beispielsweise eine Länge von 1900 mm bei einem Durchmesser von 19 mm aufweisen, umfassen jeweils ein Quarzentladungsrohr, das in einem Quarz- oder Duranglaszylinder 37 beabstandet angeordnet ist, so daß zwischen dem Quarzentladungsrohr und dem Duranglaszylinder 37 eine Luftschicht 38 befindet. Die Luftschicht ist dabei vorzugsweise größer als 10 mm. Um den ersten Duranglaszylinder 37 ist ein zweiter Duranglaszylinder 39 angeordnet, der über eine Strahlungskühlerfassung abgedichtet ist. Zwischen den beiden Duranglaszylindern 37 und 39 ist eine Wasserschicht 40, die über die Strahlungskühlerfassung zwischen die Zylinder ein- und ausgeleitet werden kann. Dabei bewirkt die Luftschicht 38, daß die Temperatur im Entladungsgefäß der Strahlungsquellen 10 nicht zu stark erniedrigt wird, um einen ausreichenden Dampfdruck aufrechtzuerhalten. Die Wasserschicht 40 hingegen filtert nicht erwünschte Wärmestrahlung, wobei die Durangläser die UV-Strahlung herausfiltern. Um den zweiten Duranglaszylinder 39 ist ein transparenter Kunststoffzylinder 41 angeordnet, der stimseitig über zwei O-Ring-abgedichtete Bohrungen wasserdicht in der Reflektorrinne 36 befestigt ist. Zwischen dem zweiten Duranglaszylinder 39 und dem Kunststoffzylinder 41 befindet sich dann eine zweite Luftschicht 42. Auf der Oberseite ist die Reflektorrinne 36 durch eine vorzugsweise elastische Folie 43 abgeschlossen. Die Folie 43 kann dabei auch zur topischen Sauerstoffzufuhr dienen und beispielsweise als beschichtete und mit Sauerstoff gesättigte Silikonpolymerfolie ausgebildet sein. Die Dicke der Folie 43 beträgt dabei vorzugsweise 50-100 µm, um einen ausreichend hohen Wärmeübergang vom Patienten 13 zu dem darunter befindlichen Wasser der Kühleinrichtung zu gewährleisten. Die Folie 43 wird dabei derart locker befestigt, so daß sich das Kühlwasser auf der gesamten Körperunterseite flächendeckend anschmiegt. Wegen der begrenzten mechanischen Stabilität der Folie 43 wird diese durch eine netzartige oder semitransparente Auflage mechanisch unterstützt. Zur gleichmäßigeren Bestrahlung des Patienten 13 ist zwischen den Strahlungsquellen 10 und dem Patienten 13 ein Diffusor 44 angeordnet, der weitgehend eine direkte Bestrahlung verhindert, was durch die stilisierten Strahlenverläufe angedeutet ist.

## Patentansprüche

1. Therapeutische Bestrahlungsanordnung, umfassend mindestens eine flächenhafte Strahlungsquelle, die im Bereich zwischen 315-1500 nm emittiert, mit einer Einrichtung zur topischen Sauerstoffzugabe,
**dadurch gekennzeichnet, dass**
der Einrichtung zur topischen Sauerstoffzugabe ein Ultraschall-Applikator zugeordnet ist, durch den Gewebekavitationen erzeugbar sind, wobei die Bestrahlungsanordnung zusätzlich eine Einrichtung und/oder Mittel zur inspiratorischen Sauerstoffzugabe umfasst.

2. Therapeutische Bestrahlungsanordnung nach Anspruch 1, **dadurch gekennzeichnet, dass** die Einrichtung einen gasdichten Hüllkörper (7) umfasst, in dem eine reine Sauerstoffatmosphäre erzeugbar ist.

3. Therapeutische Bestrahlungsanordnung nach Anspruch 2, **dadurch gekennzeichnet, dass** die Strahlungsquellen (10) im Hüllkörper (7) angeordnet sind.

4. Therapeutische Bestrahlungsanordnung nach einem der vorangegangenen Ansprüche, **dadurch gekennzeichnet, dass** die Einrichtung zur inspiratorischen Sauerstoffzugabe eine Nasen-Mund-Maske (18) umfasst.

5. Therapeutische Bestrahlungsanordnung nach Anspruch 4, **dadurch gekennzeichnet, dass** die Nasen-Mund-Maske (18) aus einem für die emittierte Strahlung transparenten Material besteht.

6. Therapeutische Bestrahlungsanordnung nach einem der vorangegangenen Ansprüche, **dadurch gekennzeichnet, dass** über die Einrichtung zur inspiratorischen Sauerstoffzugabe Flußraten zwischen 1-100 ml/min einstellbar sind.

7. Therapeutische Bestrahlungsanordnung nach einem der vorangegangenen Ansprüche, **dadurch gekennzeichnet, dass** die Strahlungsquellen (10) mit der Einrichtung zur inspiratorischen und/oder topischen Sauerstoffzugabe derart gekoppelt sind, daß die Strahlungsquellen (10) zeitlich versetzt zur Sauerstoffzufuhr aktivierbar sind.

8. Therapeutische Bestrahlungsanordnung nach einem der vorangegangenen Ansprüche, **dadurch gekennzeichnet, dass** die Bestrahlungsanordnung (1) eine Kühleinrichtung (11) für eine zu bestrahlende Fläche umfasst.

9. Therapeutische Bestrahlungsanordnung nach Anspruch 8, **dadurch gekennzeichnet, dass** mittels der Kühleinrichtung (11) die zu bestrahlende Fläche auf <30°C temperierbar ist.

10. Therapeutische Bestrahlungsanordnung nach einem der Ansprüche 8 oder 9, **dadurch gekennzeichnet, dass** die Kühleinrichtung (11) mit der topischen Sauerstoffzugabe zeitlich gekoppelt ist.

11. Therapeutische Bestrahlungsanordnung nach einem der vorangegangenen Ansprüche, **dadurch gekennzeichnet, dass** die Strahlungsquelle (10) im Bereich von 400-440 nm und 530-570 nm die maximale spektrale Leistungsdichte aufweist.

12. Therapeutische Bestrahlungsanordnung nach Anspruch 11, **dadurch gekennzeichnet, dass** die Strahlungsquelle (10) als Quecksilbemiederdrucklampe mit einem der nachfolgenden Leuchtstoffe Sr₂P₂O₇:Eu, (SrMg)₂P₂O₇:Eu, Sr₅Cl(PO₄)₃:Eu, BaMg₂Al₁₆O₂₇:Eu, SrMgAl₁₈O₃₉:Eu, BaMg₂Al₁₆O₂₇:Eu:Mn, Sr₃(PO₄)₂:Eu, Ba₃(PO₄)₂:Eu, CaWO₄:Pb oder CaWO₄ und einem UV-Filter ausgebildet ist.

13. Therapeutische Bestrahlungsanordnung nach Anspruch 11, **dadurch gekennzeichnet, dass** die Strahlungsquelle (10) als mindestens eine Mitteloder Hochdruckentladungslampe mit einem Zündgas und den Additiven Quecksilber und/oder Gallium und/oder Galliumjodid und/oder Indiumjodid und/oder Selen und/oder Antimon und/oder Zink und/oder Cadmium ausgebildet ist.

14. Therapeutische Bestrahlungsanordnung nach einem der vorangeganenen Ansprüche, **dadurch gekennzeichnet, dass** die Einrichtung zur topischen Sauerstoffzufuhr eine mindestens einfach beschichtete, mit Sauerstoff beladene Polymerfolie umfasst.

## Claims

1. Therapeutic irradiation arrangement, comprising at least one two-dimensional radiation source which emits in the range between 315-1500 nm, having a device for topical oxygen supply,
**characterised in that**
an ultrasonic applicator is associated with the device for topical oxygen supply, by means of which applicator tissue cavitations can be produced, the irradiation arrangement comprising in addition a device and/or means for inspiratory oxygen supply.

2. Therapeutic irradiation arrangement according to claim 1, **characterised in that** the device includes a gas-impermeable enveloping body (7) in which a pure oxygen atmosphere can be produced.

3. Therapeutic irradiation arrangement according to claim 2, **characterised in that** the radiation sources (10) are disposed in the enveloping body (7).

4. Therapeutic irradiation arrangement according to one of the preceding claims, **characterised in that** the device for inspiratory oxygen supply includes a nose-mouth mask (18).

5. Therapeutic irradiation arrangement according to claim 4, **characterised in that** the nose-mouth mask (18) is formed from a material which is transparent for the emitted radiation.

6. Therapeutic irradiation arrangement according to one of the preceding claims, **characterised in that** flow rates between 1-100 ml/min can be set via the device for inspiratory oxygen supply.

7. Therapeutic irradiation arrangement according to one of the preceding claims, **characterised in that** the radiation sources (10) are coupled with the device for inspiratory and/or topical oxygen supply in such a manner that the radiation sources (10) are activatable temporally offset relative to the oxygen supply.

8. Therapeutic irradiation arrangement according to one of the preceding claims, **characterised in that** the irradiation arrangement (1) includes a cooling device (11) for a surface to be irradiated.

9. Therapeutic irradiation arrangement according to claim 8, **characterised in that** the surface to be irradiated can be set at a temperature of < 30°C by means of the cooling device (11).

10. Therapeutic irradiation arrangement according to one of the claims 8 or 9, **characterised in that** the cooling device (11) is coupled temporally with the topical oxygen supply.

11. Therapeutic irradiation arrangement according to one of the preceding claims, **characterised in that** the radiation source (10) has the maximum spectral power density in the range of 400-440 nm and 530-570 nm.

12. Therapeutic irradiation arrangement according to claim 11, **characterised in that** the radiation source (10) is configured as a low-pressure mercury vapour lamp with one of the subsequent fluorescent materials
Sr₂P₂O₇:Eu, (SrMg)₂P₂O₇:Eu, Sr₅Cl(PO₄)₃:Eu, BaMg₂Al₁₆O₂₇:Eu, SrMgAl₁₈O₃₉:Eu, BaMg₂Al₁₆O₂₇:Eu:Mn, Sr₃(PO₄)₂:Eu, Ba₃(PO₄)₂:Eu, CaWO₄:Pb or CaWO₄ and a UV filter.

13. Therapeutic irradiation arrangement according to claim 11, **characterised in that** the radiation source (10) is configured as at least a medium-pressure or high-pressure discharge lamp with an ignition gas and the additives mercury and/or gallium and/or gallium iodide and/or indium iodide and/or selenium and/or antimony and/or zinc and/or cadmium.

14. Therapeutic irradiation arrangement according to one of the preceding claims, **characterised in that** the device for topical oxygen supply includes a polymer film which is at least coated on one side and laden with oxygen.

## Revendications

1. Dispositif de rayonnement thérapeutique comprenant au moins une source de rayonnement de surface qui émet dans la zone comprise entre 315-1500 nm, avec une installation d'addition d'oxygène topique,
**caractérisé en ce que**,
un applicateur d'ultrasons est attribué à l'installation d'addition d'oxygène topique, par lequel peuvent être produites des cavitations de tissu, moyennant quoi le dispositif de rayonnement comprend en sus une installation et/ou des moyens d'addition d'oxygène inspiratoire.

2. Dispositif de rayonnement thérapeutique selon la revendication 1, **caractérisé en ce que** l'installation comprend un corps enveloppant (7) étanche aux gaz dans lequel peut être produit une atmosphère pure en oxygène.

3. Dispositif de rayonnement thérapeutique selon la revendication 2, **caractérisé en ce que** les sources de rayonnement (10) sont disposées dans le corps enveloppant (7).

4. Dispositif de rayonnement thérapeutique selon l'une quelconque des revendications précédentes, **caractérisé en ce que** l'installation d'addition d'oxygène inspiratoire comprend un masque nez-bouche (18).

5. Dispositif de rayonnement thérapeutique selon la revendication 4, **caractérisé en ce que** le masque nez-bouche (18) se compose d'un matériau transparent pour le rayonnement émis.

6. Dispositif de rayonnement thérapeutique selon l'une quelconque des revendications précédentes, **caractérisé en ce que** des débits entre 1-100 ml/min peuvent être réglé via l'installation d'addition d'oxygène inspiratoire.

7. Dispositif de rayonnement thérapeutique selon l'une quelconque des revendications précédentes, **caractérisé en ce que** les sources de rayonnement (10) sont couplées à l'installation d'addition d'oxygène inspiratoire et/ou topique, de sorte que les sources de rayonnement (10) peuvent être activées de manière décalée dans le temps par rapport à l'alimentation en oxygène.

8. Dispositif de rayonnement thérapeutique selon l'une quelconque des revendications précédentes, **caractérisé en ce que** le dispositif de rayonnement (1) comprend une installation de refroidissement (11) pour une surface à traiter par rayons.

9. Dispositif de rayonnement thérapeutique selon la revendication 8, **caractérisé en ce que** la surface à traiter par rayons peut être tempérée à <30 °C au moyen de l'installation de refroidissement (11).

10. Dispositif de rayonnement thérapeutique selon l'une des revendications 8 ou 9, **caractérisé en ce que** l'installation de refroidissement (11) est couplée temporairement à l'addition d'oxygène topique.

11. Dispositif de rayonnement thérapeutique selon l'une quelconque des revendications précédentes, **caractérisé en ce que** la source de rayonnement (10) présente dans la zone de 400-440 nm et 530-570 nm la puissance volumique spectrale maximale.

12. Dispositif de rayonnement thérapeutique selon la revendication 11, **caractérisé en ce que** la source de rayonnement (10) est configurée comme une lampe basse pression à arc à mercure avec une des substances luminescentes suivantes Sr₂P₂O₇:Eu, (SrMg)₂P₂O₇ :Eu, Sr₅CI(PO₄)₃:Eu, BaMg₂Al₁₆O₂₇ :Eu, SrMgAl₁₈O₃₉ :Eu, BaMg₂Al₁₆O₂₇:Eu:Mn, Sr₃(PO₄)₂:Eu, Ba₃(PO₄)₂:Eu, CaWO₄:Pb ou CaWO₄ et un filtre UV.

13. Dispositif de rayonnement thérapeutique selon la revendication 11, **caractérisé en ce que** la source de rayonnement (10) est configurée comme au moins une lampe à décharge moyenne ou haute pression avec un gaz d'allumage et l'additif tel que le mercure et/ou le gallium et/ou l'iodargyre de gallium et/ou l'iodargyre d'indium et/ou le sélénium et/ou l'antimoine et/ou le zinc et/ou le cadmium.

14. Dispositif de rayonnement thérapeutique selon l'une quelconque des revendications précédentes, **caractérisé en ce que** l'installation d'alimentation en oxygène topique comprend un film polymère chargé d'oxygène au moins simplement enduit.
